# EUROPEAN PATENT SPECIFICATION

(11) **EP 1 948 183 B1**
(45) Date of publication and mention of the grant of the patent: **18.12.2013**
(21) Application number: 06816880.6
(22) Date of filing: 13.10.2006
(51) Int. Cl.: A61K 31/52, A61K 31/34, A61K 31/047, A61K 31/198, A61K 31/403, A61K 31/40, A61P 9/00, A61P 9/04, A61P 9/10

(54) **COMPOSITIONS FOR THE TREATMENT AND PREVENTION OF HEART DISEASE AND METHODS OF USING SAME**
ZUSAMMENSETZUNGEN ZUR BEHANDLUNG UND PRÄVENTION VON HERZKRANKHEITEN UND ANWENDUNGSVERFAHREN DAFÜR
COMPOSITIONS POUR LE TRAITEMENT ET LA PRÉVENTION DE MALADIES CARDIAQUES ET PROCÉDÉS D'UTILISATION DE CELLES-CI

(30) Priority: 13.10.2005 US 726484 P
(43) Date of publication of application: 30.07.2008
(73) Proprietor: Duke University, Durham, NC 27710 (US); SYSTOMICS PHARMARCEUTICALS, INC., Emeryville CA 94608 (US)
(72) Inventor: WENT, Gregory, T., Mill Valley, CA 94941-1204 (US); STAMLER, Jonathan, S., Chapel Hill, NC 27514 (US)
(74) Representative: Warner, James Alexander
(86) International application number: PCT/US2006/040117
(87) International publication number: WO 2007/044910

(56) References cited:
- WO-A-01/17528
- WO-A-99/24038
- WO-A2-01/15673
- WO-A2-02/00210
- WO-A2-2005/027887
- WO-A2-2006/041855
- FR-A1- 2 785 501
- GB-A- 1 185 947
- US-A- 5 674 893

## Description

### FIELD OF THE INVENTION

The present invention provides pharmaceutical compositions comprising a nitric oxide generating compound and a xanthine oxidase inhibitor and their use in the treatment of heart disease including congestive heart failure and ischemic coronary disease.

### BACKGROUND OF THE INVENTION

Nitro(so)vasodilators are used in the treatment of a wide variety of cardiovascular (CV) diseases including angina a heart failure. Their mechanism of action is controversial, but involves the generation of nitric oxide-related activity. The actions of nitric oxide to improve cardiac performance are critically dependent on amounts of O₂/Reactive oxygen species (ROS) that are present. In heart failure, nitric oxide (NO) bioactivity is reduced and ROS are increased. Further, some traditional nitric oxide generating compounds like nitroglycerin can exacerbate the oxidative burden in the heart and vasculature and potentially worsen outcome.

Xanthine oxidase inhibitors (XOIs) have been used in the treatment of gout, and more recently, have been investigated for the treatment of cardiovascular diseases (including endothelial dysfunction of sickle cell disease). Allopurinol, an XOI, reduces the amount of ROS produced by xanthine oxidase and increases the amount of oxygen in the heart. However, this drug has just failed in clinical trials, when used alone.

Because of the undesirable side effects that accompany some nitro(so)vasodilators and XOls, administered separately, frequently they have limited therapeutic usefulness in the treatment of cardiovascular disease. Thus, a need exists to improve the therapeutic benefits of these drugs in the treatment of cardiovascular disease, while reducing or eliminating the undesirable side effects.

WO2005027887 describes methods, compositions and uses comprising xanthine oxidase inhibitors to treat and/or prevent coronary artery disease or coronary heart disease, and related diseases or conditions. WO0200210 describes a method for treating and preventing hypertension or treating coronary heart disease by administering an agent capable or reducing uric acid levels in a patient in need of such treatment. WO0117528 describes methods of treating and preventing mortality associated with heart failure in an African American patient with hypertension by administering a therapeutically effective amount of at least one hydralazine compounds and at least one of isosorbide dinitrate and isosorbide mononitrate, and, optionally, one or more compounds, such as, for example, a digitalis, a diuretic compound, or a compound used to treat cardiovascular diseases.

### SUMMARY OF THE INVENTION

The invention provides a pharmaceutical composition including one or more xanthine oxidase inhibitors and one or more nitric oxide generating compounds, wherein the nitric oxide generating compounds do not interact with aldehyde dehydrogenase in a manner which increases oxidative stress or the nitric oxide generating compounds enhance the activity of or the expression of neuronal nitric oxide synthase. In the pharmaceutical composition of the invention, the one or more xanthine oxidase inhibitors are allopurinol or oxypurinol.

In the pharmaceutical composition of the invention, the allopurinol is admiriistered at a dose from about 1 mg/day to about 800 mg/day. In another embodiment of the pharmaceutical composition of the invention, the allopurinol is administered at a dose from about 1 mg/day to about 600 mg/day.

In the pharmaceutical composition of the invention, the one or more nitric oxide generating compounds is a member of the group consisting of isosorbide dinitrate, isosorbide mononitrate, pentaerythritol dinitrate, and pentaerythritol mononitrate. In one aspect of the invention, the isosorbide dinitrate is administered at a dose from about 1 mg/day to about 40 mg/day. In another aspect of the invention, the isosorbide dinitrate is administered at a dose from about 1 mg/day to about 20 mg/day.

In another embodiment of the pharmaceutical composition of the invention, the one or more xanthine oxidase inhibitors and the one or more nitric oxide generating compounds is administered orally.

The invention also provides compositions for use in a method of treating or preventing a cardiac pathology in a subject in need thereof, the method comprising administering to the subject a pharmaceutically acceptable dose of one or more xanthine oxidase inhibitors and one or more nitric oxide generating compounds, wherein the nitric oxide generating compounds do not interact with aldehyde dehydrogenase in a manner which increases oxidative stress or the nitric oxide generating compounds enhance the activity of or the expression of neuronal nitric oxide synthase, thereby treating or preventing the cardiac pathology in the subject in need thereof. In one embodiment, the cardiac pathology is congestive heart failure or ischemic coronary disease.

In another embodiment, the subject is a mammal. In one aspect of this embodiment, the mammal is a human.

In the compositions for use in the method of treating or preventing a cardiac pathology in a subject in need thereof, the one or more xanthine oxidase inhibitors are allopurinol or oxypurinol. In one aspect of this embodiment, the pharmaceutically effective dose of allopurinol is from about 1 mg/day to about 800 mg/day. In another aspect of this embodiment, the pharmaceutically effective dose of allopurinol is from about 1 mg/day to about 600 mg/day.

In the composition for use in the method of treating or preventing a cardiac pathology in a subject in need thereof, the one or more nitric oxide generating compounds is a member of the group consisting of isosorbide dinitrate, isosorbide mononitrate, pentaerythritol dinitrate and pentaerythritol mononitrate. In one aspect of this embodiment, the pharmaceutically effective dose of isosorbide dinitrate is from about 1 mg/day to about 40 mg/day. In another aspect of this embodiment, the pharmaceutically effective dose of isosorbide dinitrate is from about 1 mg/day to about 20 mg/day.

In another embodiment, the one or more xanthine oxidase inhibitors and the one or more nitric oxide generating compounds are administered orally.

In another embodiment, the subject is of Asian descent. In another embodiment, the subject is diabetic or is suffering from a lung disease.

The invention also provides a kit for use in treating or preventing a cardiac pathology in a subject in need thereof, according to claim 13.

In one embodiment of the kit, the one or more xanthine oxidase inhibitors is allopurinol and the one or more nitric oxide generating compounds is isosorbide dinitrate.

### DETAILED DESCRIPTION OF THE INVENTION

The invention is based on the improved treatment of cardiovascular disease by the combination therapy of NO generating compounds, which bypass the aldehyde dehydrogenase (ALDH-2) pathway of bioactivation or are targeted to the sarcoplasmic reticulum of cardiac muscle cells, and xanthine oxidase inhibitors (XOIs).

It has been shown that the bioactivation (*i*.*e*. the release of NO) of nitroglycerin occurs in an ALDH-2 dependent manner. (Kollau et al. Biochem J. 385:769-777 (2005)). Nitroglycerin is converted to 1,2 glycerol dinitrate and nitrite, by ALDH-2, which is believed to be involved in conversion to NO. *In vitro* studies have shown that the combination of nitroglycerin with ALDH-2 leads to the activation of soluble guanylate cyclase, which is believed to be activated by NO or an associated factor.

ALDH-2 functions as a protector against oxidative stress. Thus, it is desirable for the mitochondrial biotransformation of NO generating compounds by aldehyde dehydrogenase (ALDH-2) to be circumvented to avoid oxidative stress through saturation of ALDH-2 capacity. This is particularly true in the case of patient subsets with increased mitochondrial oxidant burden (*e*.*g*. diabetics, and patients with lung disorders such as asthma, chronic obstructive pulmonary disorder (COPD) (which collectively encompasses bronchitis and emphysema), adult respiratory distress syndrome (ARDS), infant respiratory distress syndrome/ bronchopulmonary dysplasia, lung cancer, cystic fibrosis especially during exacerbations, idiopathic pulmonary fibrosis, pneumonia, lung transplantation, bronchopulmonary dysplasia, mineral dust pneumoconiosis, and radiation toxicity) and/or decreased ALDH-2 activity (*e*.*g*. Asians). (Ohta, et al. Ann N Y Acad Sci. 1011:36-44 (Apr. 2004)).

Repletion of NO, especially in an ALDH-2 independent and sarcoplasmic reticulum targeted manner, will enable the benefits of XOIs. It has been shown that deficiency in the expression of nNOS, located in the sarcoplasmic reticulum, leads to the activation of xanthine oxidase. (Kahn et al. PNAS USA 101(45):15944-48 (Nov. 9, 2004). Thus, the beneficial effects of NO/nitrates also depend on the site and mechanism of drug biotransformation. Targeting NO to its site of action (*e*.*g*. the sarcoplasmic reticulum) is beneficial for the avoidance of nitrosative stress.

Thus, it is desirable for NO generating compounds useful in the combination therapy of the invention to target NO delivery as a means to enhance XO inhibition either through enhancement of ALDH-2 capacity or NO production in the sarcoplasmic reticulum. NO generating agents of the invention include compounds that circumvent mitochondrial aldehyde dehydrogenase for example, isosorbide dinitrate (ISDN) or isosorbide mononitrate (ISMN).

NO generating compounds of the invention also include pentaerythritol dinitrate, and pentaerythritol mononitrate. The compounds do interact and are bioactivated by mitochondrial aldehyde dehydrogenase. However, these compounds do not cause the same increases in oxidative stress as other NO generating compounds which are bioactivated by mitochondrial aldehyde dehydrogenase (*e*.*g*. nitroglycerine). Because of this, pentaerythritol dinitrate, and pentaerythritol mononitrate are also NO generating compounds used in the combination therapies of the invention.

### Definitions

The following definitions are provided to assist the reader. Unless otherwise defined, all terms of art, notations and other scientific or medical terms or terminology used herein are intended to have the meanings commonly understood by those of skill in the chemical and medical arts. In some cases, terms with commonly understood meanings are defined herein for clarity and/or for ready reference, and the inclusion of such definitions herein should not necessarily be construed to represent a substantial difference over the definition of the term as generally understood in the art.

As used herein, "treating" a condition or patient refers to taking steps to obtain beneficial or desired results, including clinical results. For purposes of this invention, beneficial or desired clinical results include, but are not limited to, shortness of breath, persistent coughing or wheezing, edema, fatigue, lack of appetite, nausea, confusion, impaired thinking, increased heart rate, exercise intolerance, angina pectoris, myocardial infarction, and cardiac ischemia.

As used herein, "reduction" of a symptom or symptoms (and grammatical equivalents of this phrase) means decreasing of the severity or frequency of the symptom(s), or elimination of the symptom(s).

As used herein, "administering" or "administration of" a drug to a subject (and grammatical equivalents of this phrase) includes both direct administration, including self-administration, and indirect administration, including the act of prescribing a drug. For example, as used herein, a physician who instructs a patient to selfadminister a drug and/or provides a patient with a prescription for a drug is administering the drug to the patient.

As used herein, a "manifestation" of a disease refers to a symptom, sign, anatomical state, physiological state, or report characteristic of a subject with the disease.

As used herein, a "therapeutically effective amount" of a drug or agent is an amount of a drug or agent that, when administered to a subject with a disease or condition, will have the intended therapeutic effect, *e*.*g*., alleviation, amelioration, palliation or elimination of one or more manifestations of the disease or condition in the subject. The full therapeutic effect does not necessarily occur by administration of one dose and may occur only after administration of a series of doses. Thus, a therapeutically effective amount may be administered in one or more administrations.

As used herein, a "prophylactically effective amount" of a drug is an amount of a drug that, when administered to a subject, will have the intended prophylactic effect, *e*.*g*., preventing or delaying the onset (or reoccurrence) of disease or symptoms, or reducing the likelihood of the onset (or reoccurrence) of disease or symptoms. The full prophylactic effect does not necessarily occur by administration of one dose and may occur only after administration of a series of doses. Thus, a prophylactically effective amount may be administered in one or more administrations.

Administration of an agent "in combination with" includes parallel administration (administration of both the agents to the patient over a period-of time, such as administration on alternate days for one month), co-administration (in which the agents are administered at approximately the same time, *e*.*g*., within about a few minutes to a few hours of one another), and co-formulation (in which the agents are combined or compounded into a single dosage form suitable for oral or parenteral administration).

### NO Generating Compounds

NO generating compounds useful in the invention include those NO generating compounds which are not bioactivated by the ALDH-2 pathway.

### ALDH-2 independent NO generating compounds

Isosorbide dinitrate, or 1,4:3,6-dianhydrosorbitol 2,5-dinitrate (commercially available as DILLATRATE^{™} (Schwarz Pharma, Milwaukee, Wis.); ISORDIL^{™} and ISORDILR TITRADOSE^{™} (Wyeth Laboratories Inc., Philadelphia, Pa.); and SORBITRATE^{™} (Zeneca Pharmaceuticals, Wilmington, Del.)), and isosorbide mononitrate or 1,4:3,6-dianhydrosorbitol 5-mononitrate (commercially available, for example, under the trade names IMDUR^{™} (A. B. Astra, Sweden); MONOKET^{™} (Schwarz Pharma, Milwaukee, Wis.); and ISMO^{™} (Wyeth-Ayerst company, Philadephia, Pa.)) are peripheral dilators, producing a vasodilatory effect on both peripheral arteries and veins with predominant effects on the latter. Pentaerythritol dinitrate and pentaerythritol mononitrate are NO generating compound which are used in the treatment of angina. These compounds are able to generate NO without generating significant oxidative stress, despite their interaction with ALDH-2.

It has been shown that the ALDH-2 inhibitor benomyl reduced the vasodilator potency, but not the efficacy, of GTN, pentaerythritol tetranitrate (PETN), and pentaerythritol trinitrate in phenylephrine-constricted rat aorta, whereas vasodilator responses to isosorbide dinitrate, isosorbide-5-mononitrate, pentaerythritol dinitrate, pentaerythritol mononitrate, and the endothelium-dependent vasodilator acetylcholine were not affected. (Daiber et al. Mol Pharmacol. 66(6):1372-82 (Epub Aug 26, 2004)).

This suggests that isosorbide dinitrate, isosorbide mononitrate, pentaerythritol dinitrate and pentaerythritol mononitrate have an NO generating effect in a manner that does not increase oxidative stress and thus are appropriate for use in the combination therapy of the invention.

Preferably, isosorbide dinitrate is administered in an amount from about 1 mg/day to about 40 mg/day. More preferably, isosorbide dinitrate is administered in an amount from about 1 mg/day to about 20 mg/day. Isosorbide mononitrate is administered in an amount from about 1 mg/day to about 120 mg/day per day. Pentaerythritol dinitrate, pentaerythritol mononitrate may be administered in an amount from about 1 mg/day to about 1,000 mg/day. Preferably these NO generating compounds are administered orally.

### Xanthine Oxidase Inhibitors (XOI)

Xanthine oxidase inhibitors inhibit the activity of xanthine oxidase and have been used for the treatment of gout. XOIs are included in the combination therapy of the invention because of xanthine oxidase being linked to the production of superoxide, a ROS which is associated with cardiac pathology. Increases in the production of superoxide by xanthine oxidase are associated with increased oxidative stress and cardiac dysfunction.

Preferred XOIs used in the invention are allopurinol and oxypurinol. Clinical trials using allopurinol alone were not successful. The combination of allopurinol with the NO generating compounds will potentiate the therapeutic effect of allopurinol, making an effective treatment for heart disease. Preferably, allopurinol and oxypurinol are administered orally. Preferably, allopurinol and oxypurinol are administered in an amount from about 1 mg/day to about 800 mg/day. More preferably, allopurinol and oxypurinol are administered in an amount from about 100 mg/day to about 600 mg/day.

### Combination Therapies

The combination of the invention include the combination of one or more NO generating compounds, which bypass the aldehyde dehydrogenase (ALDH-2) pathway of bioactivation and one or more xanthine oxidase inhibitors (XOIs). As described above, NO generating compounds, which bypass the aldehyde dehydrogenase (ALDH-2) pathway of bioactivation include isosorbide dinitrate and isosorbide mononitrate. NO generating compounds, which use the ALDH-2 pathway, but do not increase oxidative stress include pentaerythritol dinitrate and pentaerythritol mononitrate. XOIs include allopurinol and oxypurinol. Any combination of NO generating compound or XOI may be used in the combination of the invention.

### Synergy/Additivity

Synergy is defined as the interaction of two or more agents so that their combined effect is greater than the sum of their individual effects. For example, if the effect of drug A alone in treating a disease is 25%, and the effect of drug B alone in treating a disease is 25%, but when the two drugs are combined the effect in treating the disease is 75%, the effect of A and B is synergistic.

Additivity is defined as the interaction of two or more agents so that their combined effect is greater than the average of their individual effects. For example, if the effect of drug A alone in treating a disease is 25%, and the effect of drug B alone in treating a disease is 25%, but when the two drugs are combined the effect in treating the disease is greater than 25%, the effect of A and B is additive.

An improvement in the drug therapeutic regimen can be described as the interaction of two or more agents so that their combined effect reduces the incidence of adverse event (AE) of either or both agents used in co- therapy. This reduction in the incidence of adverse effects can be a result of, *e*.*g.*, administration of lower dosages of either or both agent used in the co-therapy. For example, if the effect of Drug A alone is 25% and has an adverse event incidence of 45% at labeled dose; and the effect of Drug B alone is 25% and has an adverse event incidence of 30% at labeled dose, but when the two drugs are combined at lower than labeled doses of each, if the overall effect is 35%, and the adverse incidence rate is 20%, there is an improvement in the drug therapeutic regimen.

The combinations described above have both synergistic and additive effects in the treatment and prevention of various pathologies related to heart disease, specifically congestive heart failure and ischemic coronary disease.

### Cardiac Pathologies

The combination of the present invention can be used to treat any mammal, including humans and animals, suffering from a disease, symptom, or condition related to a cardiac disorder or heart disease. Types of heart disease include coronary artery disease (including heart attack), abnormal heart rhythms or arrythmias, heart failure, heart valve disease, congenital heart disease, heart muscle disease (cardiomyopathy), pericardial disease, aorta disease and Marfan syndrome, vascular disease (blood vessel disease), peripheral vascular disease, carotid disease, arthrosclerosis, congestive heart failure and ischemic coronary disease.

Congestive heart failure (CHF) is an imbalance in pump function in which the heart fails to maintain the circulation of blood adequately. The most severe manifestation of CHF, pulmonary edema, develops when this imbalance causes an increase in lung fluid secondary to leakage from pulmonary capillaries into the interstitium and alveoli of the lung.

Ischemic coronary disease (also known as coronary artery disease) is a condition in which fatty deposits (atheroma) accumulate in the cells lining the wall of the coronary arteries. These fatty deposits build up gradually and irregularly in the large branches of the two main coronary arteries which encircle the heart and are the main source of its blood supply. This process is called atherosclerosis which leads to narrowing or hardening of the blood vessels supplying blood to the heart muscle (the coronary arteries). This results in ischemia (inability to provide adequate oxygen) to heart muscle and this can cause damage to the heart muscle . Complete occlusion of the blood vessel leads to a heart attack (myocardial infarction). In the United States, cardiovascular disease is the leading cause of death among both sexes, and ischemic coronary disease is the commonest cause of cardiovascular disease.

The combinations of the invention are useful in the prevention and treatment of any of the forms of heart disease described above, and especially congestive heart failure and ischemic coronary disease. The combinations of the invention are particularly useful in the treatment of cardiac pathologies in patients who have high oxidative burden. Examples of patients who have high oxidative burden include diabetics, patients with lung ailments, patients with sickle cell anemia and patients of Asian descent.

### Pharmaceutical Compositions, Dosing and Administration

The NO generating compounds, which bypass the aldehyde dehydrogenase (ALDH-2) pathway of bioactivation and xanthine oxidase inhibitors (XOIs) are administered separately or co-formulated in a suitable co-formulated dosage form. These compounds are administered to a patient in the form of a pharmaceutically acceptable salt or in a pharmaceutical composition. A compound that is administered in a pharmaceutical composition is mixed with a suitable carrier or excipient such that a therapeutically effective amount is present in the composition. The term "therapeutically effective amount" refers to an amount of the compound that is necessary to achieve a desired endpoint (e.g., decreasing symptoms associated with heart disease).

A variety of preparations can be used to formulate pharmaceutical compositions. Techniques for formulation and administration may be found in "Remington: The Science and Practice of Pharmacy, Twentieth Edition," Lippincott Williams & Wilkins, Philadelphia, PA. Tablets, capsules, pills, powders, granules, dragees, gels, slurries, ointments, solutions, suppositories, injections, inhalants and aerosols are examples of such formulations. The formulations can be administered in either a local or systemic manner or in a depot or sustained release fashion. Administration of the composition can be performed in a variety of ways. The compositions and combination therapies of the invention may be administered in combination with a variety of pharmaceutical excipients, including stabilizing agents, carriers and/or encapsulation formulations as described herein.

The preparation of pharmaceutical or pharmacological compositions will be known to those of skill in the art in light of the present disclosure. Typically, such compositions may be prepared as injectables, either as liquid solutions or suspensions; solid forms suitable for solution in, or suspension in, liquid prior to injection; as tablets or other solids for oral administration; as time release capsules; or in any other form currently used, including creams, lotions, mouthwashes, inhalants and the like.

For human administration, preparations should meet sterility, pyrogenicity, general safety and purity standards as required by the FDA.

Administration of thioesterification agents alone or in combination therapies may be, *e*.*g*., subcutaneous, intramuscular or intravenous injection, or any other suitable route of administration. A particularly convenient frequency for the administration of the compounds of the invention is once a day.

Upon formulation, therapeutics will be administered in a manner compatible with the dosage formulation, and in such amount as is pharmacologically effective. The formulations are easily administered in a variety of dosage forms, such as the injectable solutions described, but drug release capsules and the like can also be employed. In this context, the quantity of active ingredient and volume of composition to be administered depends on the host animal to be treated. Precise amounts of active compound required for administration depend on the judgment of the practitioner and are peculiar to each individual.

A minimal volume of a composition required to disperse the active compounds is typically utilized. Suitable regimes for administration are also variable, but would be typified by initially administering the compound and monitoring the results and then giving further controlled doses at further intervals.

A carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Suitable preservatives for use in solution include benzalkonium chloride, benzethonium chloride, chlorobutanol, thimerosal and the like. Suitable buffers include boric acid, sodium and potassium bicarbonate, sodium and potassium borates, sodium and potassium carbonate, sodium acetate, sodium biphosphate and the like, in amounts sufficient to maintain the pH at between about pH 6 and pH 8, and preferably, between about pH 7 and pH 7.5. Suitable tonicity agents are dextran 40, dextran 70, dextrose, glycerin, potassium chloride, propylene glycol, sodium chloride, and the like, such that the sodium chloride equivalent of the ophthalmic solution is in the range 0.9 plus or minus 0.2%. Suitable antioxidants and stabilizers include sodium bisulfite, sodium metabisulfite, sodium thiosulfite, thiourea and the like. Suitable wetting and clarifying agents include polysorbate 80, polysorbate 20, poloxamer 282 and tyloxapol. Suitable viscosity-increasing agents include dextran 40, dextran 70, gelatin, glycerin, hydroxyethylcellulose, hydroxmethylpropylcellulose, lanolin, methylcellulose, petrolatum, polyethylene glycol, polyvinyl alcohol, polyvinylpyrrolidone, carboxymethylcellulose and the like.

The thioesterification agents and combination therapies of the invention can be formulated by dissolving, suspending or emulsifying in an aqueous or nonaqueous solvent. Vegetable (*e*.*g*., sesame oil, peanut oil) or similar oils, synthetic aliphatic acid glycerides, esters of higher aliphatic acids and propylene glycol are examples of nonaqueous solvents. Aqueous solutions such as Hank's solution, Ringer's solution or physiological saline buffer can also be used. In all cases the form must be sterile and must be fluid to the extent that easy syringability exists. It must be stable under the conditions of manufacture and storage and must be preserved against the contaminating action of microorganisms, such as bacteria and fungi.

Solutions of active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly, concentrated solutions for subcutaneous or intramuscular injection is also contemplated. In this regard, the use of DMSO as solvent is preferred as this will result in extremely rapid penetration, delivering high concentrations of the active compound(s) or agent(s) to a small area.

Where one or both active ingredients is given orally, it can be formulated through combination with pharmaceutically acceptable carriers that are well known in the art. The carriers enable the compound to be formulated, for example, as a tablet, pill, capsule, solution, suspension, sustained release formulation; powder, liquid or gel for oral ingestion by the patient. Oral use formulations can be obtained in a variety of ways, including mixing the compound with a solid excipient, optionally grinding the resulting mixture, adding suitable auxiliaries and processing the granule mixture. The following list includes examples of excipients that can be used in an oral formulation: sugars such as lactose, sucrose, mannitol or sorbitol; cellulose preparations such as maize starch, wheat starch, potato starch, gelatin, gum tragacanth, methyl cellulose, hydroxypropylmethylcellulose, sodium carboxymethylcellulose and polyvinylpyrrolidone (PVP). Oral formulations include such normally employed excipients as, for example, pharmaceutical grades of mannitol, lactose, starch, magnesium stearate, sodium saccharine, cellulose, magnesium carbonate and the like.

In certain defined embodiments, oral pharmaceutical compositions will comprise an inert diluent or assimilable edible carrier, or they may be enclosed in hard or soft shell gelatin capsule, or they may be compressed into tablets, or they may be incorporated directly with the food of the diet. For oral therapeutic administration, the active compounds may be incorporated with excipients and used in the form of ingestible tablets, buccal tables, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 75% of the weight of the unit, or preferably between 25-60%. The amount of active compounds in such therapeutically useful compositions is such that a suitable dosage will be obtained.

The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup of elixir may contain the active compounds sucrose as a sweetening agent methyl and propylparabensas preservatives, a dye and flavoring, such as cherry or orange flavor.

Optionally, the NO generating compounds, which bypass the aldehyde dehydrogenase (ALDH-2) pathway of bioactivation and xanthine oxidase inhibitors (XOIs) of the present invention, or both agents are prepared using the OROS® technology, described for example, in U.S. Patent Nos. 6,919,373, 6,923,800, 6,929,803, 6,939,556, and 6,930,128. This technology employs osmosis to provide precise, controlled drug delivery for up to 24 hours and can be used with a range of compounds, including poorly soluble or highly soluble drugs. OROS® technology can be used to deliver high drug doses meeting high drug loading requirements. By targeting specific areas of the gastrointestinal tract, OROS® technology may provide more efficient drug absorption and enhanced bioavailability. The osmotic driving force of OROS® and protection of the drug until the time of release eliminate the variability of drug absorption and metabolism often caused by gastric pH and motility

Additional formulations suitable for other modes of administration include suppositories. For suppositories, traditional binders and carriers may include, for example, polyalkylene glycols or triglycerides; such suppositories may be formed from mixtures containing the active ingredient in the range of 0.5% to 10%, preferably 1%-2%.

The subject is a mammal, more preferably a human. The following properties or applications will essentially be described for humans although they may also be applied to non-human mammals, *e*.*g*., apes, monkeys, dogs, mice, etc. The invention therefore can also be used in a veterinarian context. **Kits**

The invention further relates to kits containing one or more NO generating compounds; which bypass the aldehyde dehydrogenase (ALDH-2) pathway of bioactivation and one or more xanthine oxidase inhibitors (XOIs), in separate containers. Optionally the kit further includes instructions for use of the kit. The kit may be for use in the treatment or prevention of cardiac diseases such as congestive heart failure or ischemic coronary disease.

The following examples are nonlimiting and meant only to illustrate various aspects of the invention.

### EXAMPLES

### EXAMPLE 1. Treatment with Allopurinol and Isosorbide Dinitrate.

In this example, a qualified animal model for cardiac failure is employed to examine the dose ranges of synergistic interaction of NO generating compounds and xanthine oxidase inhibitors.

### Animal Models and methods

**Animal Model**: Heart failure was modeled in rats through the injection of 300 mg/kg of isoproteronol. Rats are administered allopurinol and/or isosorbide dinitrate, as detailed below for three months prior to injection with isoproteronol.

**Treatment**: Cohorts are treated in 4 arms with 2-4 dose ranges of each drug and a placebo, at a compensated dose for animal size, metabolism and circulation, or about 1/6 the mg/kg equivalence. Arm 1: saline, Arm 2: allopurinol; Arm 3: isosorbide dinitrate; Arm 4: allopurinol plus isosorbide dinitrate. These arms are repeated at 2 dose ranges of both allopurinol and isosorbide dinitrate to measure the dose response relationship.

**Study Assessment:** Animals are assessed for left ventricular (LV) end-diastolic pressure, peak-negative dP/dt and LV ejection fraction. A histological analysis is also made of the rat hearts.

**Results:** Rats taking both allopurinol and isosorbide dinitrate display decreased left ventricular (LV) end-diastolic pressure, decreased peak-negative dP/dt and LV ejection fraction, increased peak-negative dP/dt and increased LV ejection fraction. Rats taking both allopurinol and isosorbide dinitrate show results consistent with rapid cardiac improvement from heart failure and reduced cardiac damage from heart failure.

### EXAMPLE 2. Treatment with Oxypurinol and Isosorbide Dinitrate.

In this example, a qualified animal model for cardiac failure is employed to examine the dose ranges of synergistic interaction of NO generating compounds and xanthine oxidase inhibitors.

### Animal Models and methods

**Animal Model:** Heart failure was modeled in rats through the injection of 300 mg/kg of isoproteronol. Rats are administered oxypurinol and/or isosorbide dinitrate, as detailed below for three months prior to injection with isoproteronol.

**Treatment:** Cohorts are treated in 4 arms with 2-4 dose ranges of each drug and a placebo, at a compensated dose for animal size, metabolism and circulation, or about 1/6 the mg/kg equivalence. Arm 1: saline, Arm 2: oxypurinol; Arm 3: isosorbide dinitrate; Arm 4: oxypurinol plus isosorbide dinitrate. These arms are repeated at 2 dose ranges of both oxypurinol and isosorbide dinitrate to measure the dose response relationship.

**Study Assessment:** Animals are assessed for left ventricular (LV) end-diastolic pressure, peak-negative dP/dt and LV ejection fraction. A histological analysis is also made of the rat hearts.

**Results:** Rats taking both oxypurinol and isosorbide dinitrate display decreased left ventricular (LV) end-diastolic pressure, decreased peak-negative dP/dt and LV ejection fraction, increased peak-negative dP/dt and increased LV ejection fraction. Rats taking both oxypurinol and isosorbide dinitrate show results consistent with rapid cardiac improvement from heart failure and reduced cardiac damage from heart failure.

### EXAMPLE 3: In vivo method for determining optimal steady-state concentration ratio (C_{ratio,ss})

A dose ranging study is performed using, for example, the vascular model (see, for example, Petty et al. Eur J Pharmacol 336: 127-36, 1997), the neurogenic model (see, for example, Petty et al. *supra*)*,* the murine cutaneous allodynia model (see, for example, Ghelardini et al., J. Pain 5: 413-9, 2004), and the murine hyperalgesia model (see, for example, Galeotti et al., Pharmacol. Res. 46: 245-50, 2002). An isobolic experiment ensues in which the drugs are combined in fractions of their EDXXs to add up to ED100 (e.g., ED50:ED50 or ED25:ED75). The plot of the data is constructed. The experiment points that lie below the straight line between the ED50 points on the graph are indicative of synergy, points on the line are indicative of additive effects, and points above the line are indicative of inhibitory effects. The point of maximum deviation from the isobolic line is the optimal ratio. This is the optimal steady state ratio (Cratio,ss) and is adjusted based upon the agents halflife. Similar protocols may be applied in a wide variety of validated animal models.

### EXAMPLE 4: Combinations

Representative combination ranges and ratios are provided below for compositions of the invention. These ranges are based on the formulation strategies described herein.

**Adult Dosage and Ratios for Combination Therapy**

| | Quantity, mg/day | |
|---|---|---|
| XOI mg/day | Isosorbide dinitrate | Isosorbide mononitrate |
| Allopurinol/ 1-800 | 1-40 | 1-120 |
| Oxypurinol 1-800 | 1-40 | 1-120 |

### EXAMPLE 5: Tablet containing a combination of allopurinol and isosorbide dinitrate

An extended release dosage form for administration of allopurinol and isosorbide dinitrate is prepared as three individual compartments. Three individual compressed tablets are prepared, each having a different release profile, are encapsulated into a gelatin capsule which is then closed and sealed. The components of the three tablets are as follows.

| Component | Function | Amount per tablet |
|---|---|---|
| TABLET 1 (immediate release): | | |
| Allopurinol | Active agent | 10 mg |
| Isosorbide dinitrate | Active agent | 20 mg |
| Dicalcium phosphate dihydrate | Diluent | 26.6 mg |
| Microcrystalline cellulose | Diluent | 26.6 mg |
| Sodium starch glycolate | Disintegrant | 1.2 mg |
| Magnesium Stearate | Lubricant | 0.6 mg |

| Component | Function | Amount per tablet |
|---|---|---|
| TABLET 2 (3-5 hour release): | | |
| Allopurinol | Active agent | 10 mg |
| Isosorbide dinitrate | Active agent | 20 mg |
| Dicalcium phosphate dihydrate | Diluent | 26.6 mg |
| Microcrystalline cellulose | Diluent | 26.6 mg |
| Sodium starch glycolate | Disintegrant | 1.2 mg |
| Magnesium Stearate | Lubricant | 0.6 mg |
| Eudragit RS30D | Delayed release | 4.76 mg |
| Talc | Coating component | 3.3 mg |
| Triethyl citrate | Coating component | 0.95 mg |

| Component | Function | Amount per tablet |
|---|---|---|
| TABLET 3 (Release delayed 7-10 hours): | | |
| Allopurinol | Active agent | 10 mg |
| Isosorbide dinitrate | Active agent | 20 mg |
| Dicalcium phosphate dihydrate | Diluent | 26.6 mg |
| Microcrystalline cellulose | Diluent | 26.6 mg |
| Sodium starch glycolate | Disintegrant | 1.2 mg |
| Magnesium Stearate | Lubricant | 0.6 mg |
| Eudragit RS30D | Delayed release | 6.5 mg |
| Talc | Coating component | 4.4 mg |
| Triethyl citrate | Coating component | 1.27 mg |

The tablets are prepared by wet granulation of the individual drug particles and other core components as may be done using a fluid-bed granulator, or are prepared by direct compression of the admixture of components. Tablet 1 is an immediate release dosage form, releasing the active agents within 1-2 hours following administration. Tablets 2 and 3 are coated with the delayed release coating material as may be carried out using conventional coating techniques such as spray-coating or the like. The specific components listed in the above tables may be replaced with other functionally equivalent components, e.g., diluents, binders, lubricants, fillers, coatings, and the like.

Oral administration of the capsule to a patient will result in a release profile having three pulses, with initial release of allopurinol and isosorbide dinitrate from the first tablet being substantially immediate, release of the allopurinol and isosorbide dinitrate from the second tablet occurring 3-5 hours following administration, and release of the allopurinol and isosorbide dinitrate from the third tablet occurring 7-9 hours following administration.

## Claims

1. A pharmaceutical composition comprising one or more xanthine oxidase inhibitors selected from allopurinol, oxypurinol, or pharmaceutically acceptable salts thereof, and one or more nitric oxide generating compounds selected from isosorbide dinitrate, isosorbide mononitrate, pentaerythritol dinitrate and pentaerythritol mononitrate, or pharmaceutically acceptable salts thereof.

2. A xanthine oxidase inhibitor for use in a method of treating or preventing a cardiac pathology in a subject in need thereof, the method comprising administering to the subject a pharmaceutically acceptable dose of one or more xanthine oxidase inhibitors and one or more nitric oxide generating compounds, wherein the xanthine oxidase inhibitors are selected from allopurinol, oxypurinol, or pharmaceutically acceptable salts thereof, and the nitric oxide generating compounds are selected from isosorbide dinitrate, isosorbide mononitrate, pentaerythritol dinitrate and pentaerythritol mononitrate, or pharmaceutically acceptable salts thereof.

3. A nitric oxide generating compound for use in a method of treating or preventing a cardiac pathology in a subject in need thereof, the method comprising administering to the subject a pharmaceutically acceptable dose of one or more xanthine oxidase inhibitors and one or more nitric oxide generating compounds, wherein the xanthine oxidase inhibitors are selected from allopurinol, oxypurinol, or pharmaceutically acceptable salts thereof, and the nitric oxide generating compounds are selected from isosorbide dinitrate, isosorbide mononitrate, pentaerythritol dinitrate and pentaerythritol mononitrate, or pharmaceutically acceptable salts thereof.

4. Use of a xanthine oxidase inhibitor in the manufacture of a medicament for treating or preventing a cardiac pathology in a subject in need thereof comprising administering to the subject a pharmaceutically acceptable dose of one or more xanthine oxidase inhibitors and one or more nitric oxide generating compounds, wherein the xanthine oxidase inhibitors are selected from allopurinol, oxypurinol, or pharmaceutically acceptable salts thereof, and the nitric oxide generating compounds are selected from isosorbide dinitrate, isosorbide mononitrate, pentaerythritol dinitrate and pentaerythritol mononitrate, or pharmaceutically acceptable salts thereof.

5. Use a nitric oxide generating compound in the manufacture of a medicament for treating or preventing a cardiac pathology in a subject in need thereof comprising administering to the subject a pharmaceutically acceptable dose of one or more xanthine oxidase inhibitors and one or more nitric oxide generating compounds, wherein the xanthine oxidase inhibitors are selected from allopurinol, oxypurinol, or pharmaceutically acceptable salts thereof, and the nitric oxide generating compounds are selected from isosorbide dinitrate, isosorbide mononitrate, pentaerythritol dinitrate and pentaerythritol mononitrate, or pharmaceutically acceptable salts thereof.

6. The xanthine oxidase inhibitor for use according to claim 2, the nitric oxide generating compound for use according to claim 3, or the use according to claim 4 or claim 5, wherein the cardiac pathology is congestive heart failure or ischemic coronary disease.

7. The xanthine oxidase inhibitor for use according to claim 2, the nitric oxide generating compound for use according to claim 3, or the use according to claim 4 or claim 5, wherein the subject is selected from the group consisting of a mammal, a human, a subject of Asian descent, a diabetic or a subject suffering from lung disease.

8. The pharmaceutical composition according to claim 1, the xanthine oxidase inhibitor for use according to claim 2, the nitric oxide generating compound for use according to claim 3, or the use according to claim 4 or claim 5, wherein the one or more xanthine oxidase inhibitors are allopurinol or a pharmaceutically acceptable salt thereof.

9. The pharmaceutical composition according to claim 1, the xanthine oxidase inhibitor for use according to claim 2, the nitric oxide generating compound for use according to claim 3, or the use according to claim 4 or claim 5, wherein the one or more xanthine oxidase inhibitors are allopurinol or a pharmaceutically acceptable salt thereof, and wherein the allopurinol is administered at a dose of about 1 mg/day to about 800 mg/day and preferably from about 1 mg/day to about 600 mg/day.

10. The pharmaceutical composition according to claim 1, the xanthine oxidase inhibitor for use according to claim 2, the nitric oxide generating compound for use according to claim 3, or the use according to claim 4 or claim 5, wherein the one or more nitric oxide generating compounds is isosorbide dinitrate, or a pharmaceutically acceptable salt thereof.

11. The pharmaceutical composition according to claim 1, the xanthine oxidase inhibitor for use according to claim 2, the nitric oxide generating compound for use according to claim 3, or the use according to claim 4 or claim 5, wherein the one or more xanthine oxidase inhibitors and the one or more nitric oxide generating compounds are administered orally.

12. The pharmaceutical composition according to claim 1, the xanthine oxidase inhibitor for use according to claim 2, the nitric oxide generating compound for use according to claim 3, or the use according to claim 4 or claim 5, wherein the one or more nitric oxide generating compounds is isosorbide dinitrate, or a pharmaceutically acceptable salt thereof, and wherein the isosorbide dinitrate is administered at a dose from about 1 mg/day to about 40 mg/day, and preferably from about 1 mg/day to about 20 mg/day.

13. A kit for use in treating or preventing a cardiac pathology in a subject in need thereof, said kit comprising one or more xanthine oxidase inhibitors selected from allopurinol, oxypurinol, or a pharmaceutically acceptable salt thereof, and a therapeutically effective dose of one or more nitric oxide generating compounds selected from isosorbide dinitrate, isosorbide mononitrate, pentaerythritol dinitrate and pentaerythritol mononitrate, or a pharmaceutically acceptable salt thereof, either in the same or separate packaging, and instructions for its use.

14. The pharmaceutical composition according to claim 1, the xanthine oxidase inhibitor for use according to claim 2, the nitric oxide generating compound for use according to claim 3, the use according to claim 4 or claim 5, or the kit for use according to claim 13, wherein the one or more xanthine oxidase inhibitors is allopurinol or a pharmaceutically acceptable salt thereof and the one or more nitric oxide generating compounds is isosorbide dinitrate or a pharmaceutically acceptable salt thereof.

## Patentansprüche

1. Pharmazeutische Zusammensetzung, umfassend einen oder mehrere Xanthinoxidase-Inhibitoren, die aus Allopurinol, Oxypurinol oder pharmazeutisch unbedenklichen Salzen davon ausgewählt sind, und eine oder mehrere Stickstoffmonoxid erzeugende Verbindungen, die aus Isosorbiddinitrat, Iso-sorbidmononitrat, Pentaerythritdinitrat und Pentaerythritmononitrat oder pharmazeutisch unbedenklichen Salzen davon ausgewählt sind.

2. Xanthinoxidase-Inhibitor zur Verwendung bei einer Methode zur Behandlung oder Prävention einer Herzpathologie bei einem Empfänger, bei dem diesbezüglich Bedarf besteht, bei der man dem Empfänger eine pharmazeutisch unbedenkliche Dosis eines oder mehrerer Xanthinoxidase-Inhibitoren und einer oder mehrerer Stickstoffmonoxid erzeugender Verbindungen verabreicht, wobei die Xanthinoxidase-Inhibitoren aus Allopurinol, Oxypurinol oder pharmazeutisch unbedenklichen Salzen davon ausgewählt werden und die Stickstoffmonoxid erzeugenden Verbindungen aus Isosorbiddinitrat, Isosorbidmononitrat, Pentaerythritdinitrat und Pentaerythritmononitrat oder pharmazeutisch unbedenklichen Salzen davon ausgewählt werden.

3. Stickstoffmonoxid erzeugende Verbindung zur Verwendung bei einer Methode zur Behandlung oder Prävention einer Herzpathologie bei einem Empfänger, bei dem diesbezüglich Bedarf besteht, bei der man dem Empfänger eine pharmazeutisch unbedenkliche Dosis eines oder mehrerer Xanthinoxidase-Inhibitoren und einer oder mehrerer Stickstoffmonoxid erzeugender Verbindungen verabreicht, wobei die Xanthinoxidase-Inhibitoren aus Allopurinol, Oxypurinol oder pharmazeutisch unbedenklichen Salzen davon ausgewählt werden und die Stickstoffmonoxid erzeugenden Verbindungen aus Isosorbiddinitrat, Isosorbidmononitrat, Pentaerythritdinitrat und Pentaerythritmononitrat oder pharmazeutisch unbedenklichen Salzen davon ausgewählt werden.

4. Verwendung eines Xanthinoxidase-Inhibitors bei der Herstellung eines Medikaments zur Behandlung oder Prävention einer Herzpathologie bei einem Empfänger, bei dem diesbezüglich Bedarf besteht, wobei man dem Empfänger eine pharmazeutisch unbedenkliche Dosis eines oder mehrerer Xanthinoxidase-Inhibitoren und einer oder mehrerer Stickstoffmonoxid erzeugender Verbindungen verabreicht, wobei die Xanthinoxidase-Inhibitoren aus Allopurinol, Oxypurinol oder pharmazeutisch unbedenklichen Salzen davon ausgewählt werden und die Stickstoffmonoxid erzeugenden Verbindungen aus Isosorbiddinitrat, Isosorbidmononitrat, Pentaerythritdinitrat und Pentaerythritmononitrat oder pharmazeutisch unbedenklichen Salzen davon ausgewählt werden.

5. Verwendung einer Stickstoffmonoxid erzeugenden Verbindung bei der Herstellung eines Medikaments zur Behandlung oder Prävention einer Herzpathologie bei einem Empfänger, bei dem diesbezüglich Bedarf besteht, wobei man dem Empfänger eine pharmazeutisch unbedenkliche Dosis eines oder mehrerer Xanthinoxidase-Inhibitoren und einer oder mehrerer Stickstoffmonoxid erzeugender Verbindungen verabreicht, wobei die Xanthinoxidase-Inhibitoren aus Allopurinol, Oxypurinol oder pharmazeutisch unbedenklichen Salzen davon ausgewählt werden und die Stickstoffmonoxid erzeugenden Verbindungen aus Isosorbiddinitrat, Isosorbidmononitrat, Pentaerythritdinitrat und Pentaerythritmononitrat oder pharmazeutisch unbedenklichen Salzen davon ausgewählt werden.

6. Xanthinoxidase-Inhibitor zur Verwendung nach Anspruch 2, Stickstoffmonoxid erzeugende Verbindung zur Verwendung nach Anspruch 3 oder Verwendung nach Anspruch 4 oder Anspruch 5, wobei es sich bei der Herzpathologie um dekompensierte Herzinsuffizienz oder ischämische Herzkrankheit handelt.

7. Xanthinoxidase-Inhibitor zur Verwendung nach Anspruch 2, Stickstoffmonoxid erzeugende Verbindung zur Verwendung nach Anspruch 3 oder Verwendung nach Anspruch 4 oder Anspruch 5, wobei der Empfänger aus der Gruppe bestehend aus einem Säugetier, einem Menschen, einem Empfänger asiatischer Abstammung, einem Diabetiker oder einem an Lungenkrankheit leidenden Empfänger ausgewählt ist.

8. Pharmazeutische Zusammensetzung nach Anspruch 1, Xanthinoxidase-Inhibitor zur Verwendung nach Anspruch 2, Stickstoffmonoxid erzeugende Verbindung zur Verwendung nach Anspruch 3 oder Verwendung nach Anspruch 4 oder Anspruch 5, wobei es sich bei dem einen oder den mehreren Xanthinoxidase-Inhibitoren um Allopurinol oder ein pharmazeutisch unbedenkliches Salz davon handelt.

9. Pharmazeutische Zusammensetzung nach Anspruch 1, Xanthinoxidase-Inhibitor zur Verwendung nach Anspruch 2, Stickstoffmonoxid erzeugende Verbindung zur Verwendung nach Anspruch 3 oder Verwendung nach Anspruch 4 oder Anspruch 5, wobei es sich bei dem einen oder den mehreren Xanthinoxidase-Inhibitoren um Allopurinol oder ein pharmazeutisch unbedenkliches Salz davon handelt und wobei das Allopurinol in einer Dosis von etwa 1 mg/Tag bis etwa 800 mg/Tag und vorzugsweise von etwa 1 mg/Tag bis etwa 600 mg/Tag verabreicht wird.

10. Pharmazeutische Zusammensetzung nach Anspruch 1, Xanthinoxidase-Inhibitor zur Verwendung nach Anspruch 2, Stickstoffmonoxid erzeugende Verbindung zur Verwendung nach Anspruch 3 oder Verwendung nach Anspruch 4 oder Anspruch 5, wobei es sich bei der einen oder den mehreren Stickstoffmonoxid erzeugenden Verbindungen um Isosorbiddinitrat oder ein pharmazeutisch unbedenkliches Salz davon handelt.

11. Pharmazeutische Zusammensetzung nach Anspruch 1, Xanthinoxidase-Inhibitor zur Verwendung nach Anspruch 2, Stickstoffmonoxid erzeugende Verbindung zur Verwendung nach Anspruch 3 oder Verwendung nach Anspruch 4 oder Anspruch 5, wobei der eine oder die mehreren Xanthinoxidase-Inhibitoren und die eine oder die mehreren Stickstoffmonoxid erzeugende/n Verbindungen oral verabreicht werden.

12. Pharmazeutische Zusammensetzung nach Anspruch 1, Xanthinoxidase-Inhibitor zur Verwendung nach Anspruch 2, Stickstoffmonoxid erzeugende Verbindung zur Verwendung nach Anspruch 3 oder Verwendung nach Anspruch 4 oder Anspruch 5, wobei es sich bei der einen oder den mehreren Stickstoffmonoxid erzeugenden Verbindungen um Isosorbiddinitrat oder ein pharmazeutisch unbedenkliches Salz davon handelt und wobei das Isosorbiddinitrat in einer Dosis von etwa 1 mg/Tag bis etwa 40 mg/Tag und vorzugsweise von etwa 1 mg/Tag bis etwa 20 mg/Tag verabreicht wird.

13. Kit zur Verwendung bei der Behandlung oder Prävention einer Herzpathologie bei einem Empfänger, bei dem diesbezüglich Bedarf besteht, wobei das Kit einen oder mehrere Xanthinoxidase- Inhibitoren, die aus Allopurinol, Oxypurinol oder pharmazeutisch unbedenklichen Salzen davon ausgewählt sind, und eine therapeutisch wirksame Dosis einer oder mehrerer Stickstoffmonoxid erzeugender Verbindungen, die aus Isosorbiddinitrat, Isosorbidmononitrat, Pentaerythritdinitrat und Pentaerythritmononitrat oder pharmazeutisch unbedenklichen Salzen davon ausgewählt sind, entweder in der gleichen Verpackung oder in separater Verpackung und eine Gebrauchsanweisung umfasst.

14. Pharmazeutische Zusammensetzung nach Anspruch 1, Xanthinoxidase-Inhibitor zur Verwendung nach Anspruch 2, Stickstoffmonoxid erzeugende Verbindung zur Verwendung nach Anspruch 3, Verwendung nach Anspruch 4 oder Anspruch 5 oder Kit zur Verwendung nach Anspruch 13, wobei es sich bei dem einen oder den mehreren Xanthinoxidase-Inhibitoren um Allopurinol oder ein pharmazeutisch unbedenkliches Salz davon handelt und es sich bei der einen oder den mehreren Stickstoffmonoxid erzeugenden Verbindungen um Isosorbiddinitrat oder ein pharmazeutisch unbedenkliches Salz davon handelt.

## Revendications

1. Composition pharmaceutique comprenant un ou plusieurs inhibiteurs de la xanthine oxydase choisis parmi l'allopurinol, l'oxypurinol, ou les sels pharmaceutiquement acceptables de ceux-ci, et un ou plusieurs composés générateurs de monoxyde d'azote choisis parmi le dinitrate d'isosorbide, le mononitrate d'isosorbide, le dinitrate de pentaérythritol et le mononitrate de pentaérythritol, ou les sels pharmaceutiquement acceptables de ceux-ci.

2. Inhibiteur de la xanthine oxydase destiné à être utilisé dans un procédé de traitement ou de prévention d'une pathologie cardiaque chez un sujet en ayant besoin, le procédé comprenant l'administration au sujet d'une dose pharmaceutiquement acceptable d'un ou plusieurs inhibiteurs de la xanthine oxydase et d'un ou plusieurs composés générateurs de monoxyde d'azote, les inhibiteurs de la xanthine oxydase étant choisis parmi l'allopurinol, l'oxypurinol, ou les sels pharmaceutiquement acceptables de ceux-ci, et les composés générateurs de monoxyde d'azote étant choisis parmi le dinitrate d'isosorbide, le mononitrate d'isosorbide, le dinitrate de pentaérythritol et le mononitrate de pentaérythritol, ou les sels pharmaceutiquement acceptables de ceux-ci.

3. Composé générateur de monoxyde d'azote destiné à être utilisé dans un procédé de traitement ou de prévention d'une pathologie cardiaque chez un sujet en ayant besoin, le procédé comprenant l'administration au sujet d'une dose pharmaceutiquement acceptable d'un ou plusieurs inhibiteurs de la xanthine oxydase et d'un ou plusieurs composés générateurs de monoxyde d'azote, les inhibiteurs de la xanthine oxydase étant choisis parmi l'allopurinol, l'oxypurinol, ou les sels pharmaceutiquement acceptables de ceux-ci, et les composés générateurs de monoxyde d'azote étant choisis parmi le dinitrate d'isosorbide, le mononitrate d'isosorbide, le dinitrate de pentaérythritol et le mononitrate de pentaérythritol, ou les sels pharmaceutiquement acceptables de ceux-ci.

4. Utilisation d'un inhibiteur de la xanthine oxydase dans la fabrication d'un médicament destiné au traitement ou à la prévention d'une pathologie cardiaque chez un sujet en ayant besoin comprenant l'administration au sujet d'une dose pharmaceutiquement acceptable d'un ou plusieurs inhibiteurs de la xanthine oxydase et d'un ou plusieurs composés générateurs de monoxyde d'azote, les inhibiteurs de la xanthine oxydase étant choisis parmi l'allopurinol, l'oxypurinol, ou les sels pharmaceutiquement acceptables de ceux-ci, et les composés générateurs de monoxyde d'azote étant choisis parmi le dinitrate d'isosorbide, le mononitrate d'isosorbide, le dinitrate de pentaérythritol et le mononitrate de pentaérythritol, ou les sels pharmaceutiquement acceptables de ceux-ci.

5. Utilisation d'un composé générateur de monoxyde d'azote dans la fabrication d'un médicament destiné au traitement ou à la prévention d'une pathologie cardiaque chez un sujet en ayant besoin comprenant l'administration au sujet d'une dose pharmaceutiquement acceptable d'un ou plusieurs inhibiteurs de la xanthine oxydase et d'un ou plusieurs composés générateurs de monoxyde d'azote, les inhibiteurs de la xanthine oxydase étant choisis parmi l'allopurinol, l'oxypurinol, ou les sels pharmaceutiquement acceptables de ceux-ci, et les composés générateurs de monoxyde d'azote étant choisis parmi le dinitrate d'isosorbide, le mononitrate d'isosorbide, le dinitrate de pentaérythritol et le mononitrate de pentaérythritol, ou les sels pharmaceutiquement acceptables de ceux-ci.

6. Inhibiteur de la xanthine oxydase destiné à être utilisé selon la revendication 2, composé générateur de monoxyde d'azote destiné à être utilisé selon la revendication 3, ou utilisation selon la revendication 4 ou la revendication 5, **caractérisés en ce que** la pathologie cardiaque est l'insuffisance cardiaque congestive ou une maladie coronaire ischémique.

7. Inhibiteur de la xanthine oxydase destiné à être utilisé selon la revendication 2, composé générateur de monoxyde d'azote destiné à être utilisé selon la revendication 3, ou utilisation selon la revendication 4 ou la revendication 5, **caractérisés en ce que** le sujet est choisi dans le groupe constitué par un mammifère, un être humain, un sujet d'origine asiatique, un diabétique ou un sujet atteint de maladie pulmonaire.

8. Composition pharmaceutique selon la revendication 1, inhibiteur de la xanthine oxydase destiné à être utilisé selon la revendication 2, composé générateur de monoxyde d'azote destiné à être utilisé selon la revendication 3, ou utilisation selon la revendication 4 ou la revendication 5, **caractérisés en ce que** lesdits un ou plusieurs inhibiteurs de la xanthine oxydase sont l'allopurinol ou un sel pharmaceutiquement acceptable de celui-ci.

9. Composition pharmaceutique selon la revendication 1, inhibiteur de la xanthine oxydase destiné à être utilisé selon la revendication 2, composé générateur de monoxyde d'azote destiné à être utilisé selon la revendication 3, ou utilisation selon la revendication 4 ou la revendication 5, **caractérisés en ce que** lesdits un ou plusieurs inhibiteurs de la xanthine oxydase sont l'allopurinol ou un sel pharmaceutiquement acceptable de celui-ci, et **en ce que** l'allopurinol est administré à une dose d'environ 1 mg/jour à environ 800 mg/jour et de préférence d'environ 1 mg/jour à environ 600 mg/jour.

10. Composition pharmaceutique selon la revendication 1, inhibiteur de la xanthine oxydase destiné à être utilisé selon la revendication 2, composé générateur de monoxyde d'azote destiné à être utilisé selon la revendication 3, ou utilisation selon la revendication 4 ou la revendication 5, **caractérisés en ce que** lesdits un ou plusieurs composés générateurs de monoxyde d'azote sont le dinitrate d'isosorbide, ou un sel pharmaceutiquement acceptable de celui-ci.

11. Composition pharmaceutique selon la revendication 1, inhibiteur de la xanthine oxydase destiné à être utilisé selon la revendication 2, composé générateur de monoxyde d'azote destiné à être utilisé selon la revendication 3, ou utilisation selon la revendication 4 ou la revendication 5, **caractérisés en ce que** lesdits un ou plusieurs inhibiteurs de la xanthine oxydase et lesdits un ou plusieurs composés générateurs de monoxyde d'azote sont administrés par voie orale.

12. Composition pharmaceutique selon la revendication 1, inhibiteur de la xanthine oxydase destiné à être utilisé selon la revendication 2, composé générateur de monoxyde d'azote destiné à être utilisé selon la revendication 3, ou utilisation selon la revendication 4 ou la revendication 5, **caractérisés en ce que** lesdits un ou plusieurs composés générateurs de monoxyde d'azote sont le dinitrate d'isosorbide, ou un sel pharmaceutiquement acceptable de celui-ci, et **en ce que** le dinitrate d'isosorbide est administré à une dose d'environ 1 mg/jour à environ 40 mg/jour, et de préférence d'environ 1 mg/jour à environ 20 mg/jour.

13. Kit destiné à être utilisé dans le traitement ou la prévention d'une pathologie cardiaque chez un sujet en ayant besoin, ledit kit comprenant un ou plusieurs inhibiteurs de la xanthine oxydase choisis parmi l'allopurinol, l'oxypurinol ou un sel pharmaceutiquement acceptable de ceux-ci, et une dose thérapeutiquement efficace d'un ou plusieurs composés générateurs de monoxyde d'azote choisis parmi le dinitrate d'isosorbide, le mononitrate d'isosorbide, le dinitrate de pentaérythritol et le mononitrate de pentaérythritol, ou un sel pharmaceutiquement acceptable de ceux-ci, soit dans le même emballage soit dans un emballage distinct, et les instructions pour son utilisation.

14. Composition pharmaceutique selon la revendication 1, inhibiteur de la xanthine oxydase destiné à être utilisé selon la revendication 2, composé générateur de monoxyde d'azote destiné à être utilisé selon la revendication 3, utilisation selon la revendication 4 ou la revendication 5, ou kit destiné à être utilisé selon la revendication 13, **caractérisés en ce que** lesdits un ou plusieurs inhibiteurs de la xanthine oxydase sont l'allopurinol ou un sel pharmaceutiquement acceptable de celui-ci et lesdits un ou plusieurs composés générateurs de monoxyde d'azote sont le dinitrate d'isosorbide ou un sel pharmaceutiquement acceptable de celui-ci.
